Europäisches Patentamt

European Patent Office

Office européen des brevets

(11) Publication number : **0 496 636 A1**

(12) **EUROPEAN PATENT APPLICATION**

(21) Application number : **92300628.2**

(22) Date of filing : **24.01.92**

(51) Int. Cl.⁵ : **A61F 2/46**

(30) Priority : **24.01.91 GB 9101598**

(43) Date of publication of application :
**29.07.92 Bulletin 92/31**

(84) Designated Contracting States :
**AT BE CH DE DK ES FR GB GR IT LI LU NL PT SE**

(71) Applicant : **EASCULAP LIMITED**
**43 Allen Street**
**Sheffield S3 7AX (GB)**

(72) Inventor : **Field, Richard Eddy**
**23 Redston Road**
**London N8 7HL (GB)**

(74) Representative : **Houghton, David et al**
**Hulse & Co. Cavendish Buildings West Street**
**Sheffield, S1 1ZZ (GB)**

(54) **Bone removal means.**

(57)   The invention relates to a bone removal means, and particularly a means of producing a slot in a femur that has a medial curve corresponding directly to the radius of curvature of the flare of a femoral component to be secured within the femur. This objective is met by a construction where a support means (6 ;9,2) has a slotting means (23) slidably mounted thereon, the slotting means simultaneously engaging and following a cam surface (10) located on the support means, to generate a compound movement of the slotting means that results in a slot in the bone with a curved medial surface at a required and predetermined radius of curvature.

Fig. 4

EP 0 496 636 A1

Jouve, 18, rue Saint-Denis, 75001 PARIS

This invention relates to bone removal means, and is particularly concerned with a means for the removal of cancellous bone from the femur during hip joint replacement surgery.

Ordinarily, hip joint replacement involves the positioning and securing of a femoral component to a femur to provide an artificial head to serve as ball joint for engagement with the hip. Thus, following the removal of the head of the femur at the neck connecting the head to the femur shaft the exposed end of the femur must then be prepared to enable it to receive the femoral component. The drilling of a vertical hole centrally down the femur shaft to receive a stem of the femoral component can be effected with reasonable accuracy and simplicity. However, the shaping of the interior of the femur shaft towards its head end to enable the accurate location and securing of the proximal portion of the femoral component, and ensure that the head of the femoral component is correctly disposed with the required degree of offset from the long axis of the femur for proper engagement with the hip has, hitherto, required considerable skill on the part of the surgeon, given that existing tools are hand-held and of a broach-like character driven against the cancellous bone to remove the bone adjacent the drilled vertical hole to the degree required and to create the curved surface required of the cortical bone and to be abutted by the externally curved neck of the femoral component.

The object of the present invention is to provide a bone removal means that simplifies considerably the internal shaping within the proximal end of a femur and provides a substantial guarantee that a curved surface formed within the femur shaft closely conforms to the correspondingly curved surface of the proximal region of a femoral component to be fitted within the femur shaft.

According to the present invention, a bone removal means comprises a support means, a slotting means slidably engaging said support means and further engaging a cam surface located on said support means and whereby simultaneous sliding movement of said slotting means on said support and along the cam surface combine to allow the cutting of a slot in bone with a curved medial surface at a required predetermined radius of curvature.

Thus, typically following the severing of the head of a femur and the drilling of a central hole down the femur shaft, the support means of the bone removal means can be inserted down the hole, to enable the slotting means to be brought into engagement with one side of the central hole. The relative movement permitted as between the slotting tool and the support means and controlled by the cam surface is then effected to drive the slotting means against the cancellous bone to one side of the central hole down the femur shaft to cause the removal of cancellous bone, and generate a slot extending from the central hole, the predetermined direction of the compound movement of the slotting tool being such as to generate at the medial surface a radius of curvature to correspond directly to the radius of curvature of the medial flare of the femoral component to be secured within the femur shaft.

With advantage, the slotting means may itself be a rotatable member, such as a reamer. In one form of construction, the support means may be an assembly secured to the upper end of a post formed by a tube, and extending at 90 thereto, the cam surface being formed on the assembly, and the reamer, being a solid circular section member, extends through the assembly and is provided with a bearing in engagement with the cam surface on the assembly, the lower end of the reamer being in sliding engagement with the tube. Thus, with the tube inserted down the drilled hole in the femur the bearing on the reamer can be located at the outermost end of the cam surface and the lower end of the reamer brought into contact with the top of the hole in the tube. With the reamer rotated and driven down the femur adjacent the hole, and with the bearing simultaneously moved along the cam surface, there is caused a compound arcuate movement of the reamer down the hole and when a slot is reamed extending from the hole that is parallel-sided and with a medial surface that is arcuate and of a radius of curvature dictated by the cam profile, to match the radius of curvature of the medial curve of a femoral component to be fitted within the femur shaft.

Whilst the location of the tube in the hole drilled in the femur can be sufficient to hold the support means with sufficient rigidity during reaming, it is preferred to provide additional fixing means such as by locating fixing pins on an attachment secured on the support, which fixing pins are driven into the femur surface once the tube is located in place. It is also most desirable to so position the assembly and in particular the cam surface that the axis of the slot is correctly disposed and whereby not only is the medial radius of curvature the same as that of the flare of a femoral component, but also the head of the femoral component once located on the femur is at the desired position for correct presentation of the biomechanics of the joint. Therefore, and after location of the tube in the drilled hole, a drill can be secured on the assembly towards its outer end, at a predetermined angular disposition, following which the drill is driven at that angle into the centre of the neck of the femur, to locate and secure the assembly and hence the cam surface, in the required position, and following which, the fixing pins are driven into the femur.

To facilitate the commencement of the reaming operation, after the fixing pins are driven into place, and before the drill is removed and replaced by the reamer, an osteotome (a rod and cutting blades) can be introduced down the tube to allow the ends of the blades to be driven against the top of the femur, to generate a start slot for subsequent engagement by the reamer.

One embodiment of the invention will now be described with reference to the accompanying drawings, in which:-

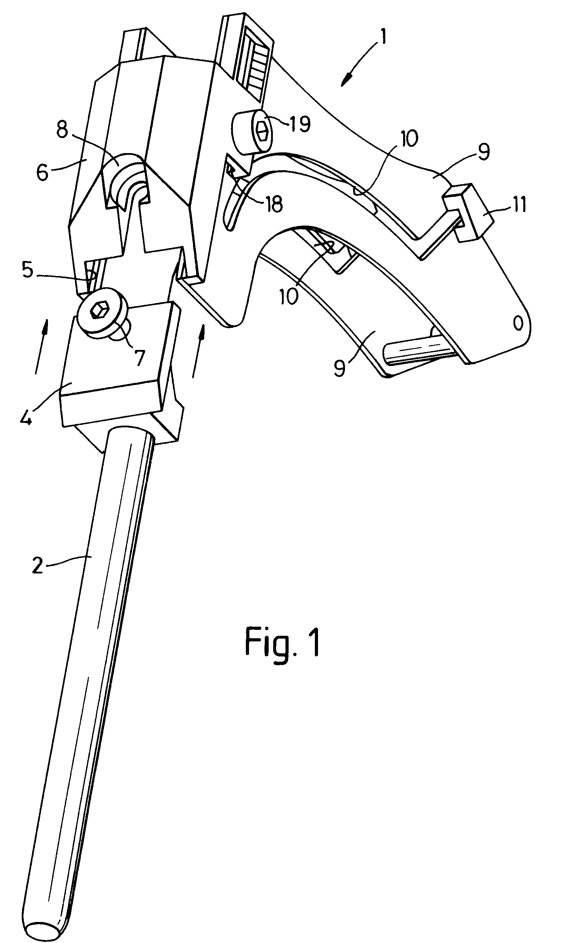

Figure 1 is a perspective view of a slotting means according to the invention;

Figure 2 is an opposite perspective view of the slotting means of Figure 1, and showing a location drill and bone fixation means in place;

Figure 3 is a sectional side elevation of the slotting means of Figure 1, showing an osteotome in place; and

Figure 4 corresponds to figure 3, but shows a reaming tool in place.

In the drawings, a bone slotting means 1 is formed by a tube 2 vertically slotted to one side at 3, and having a mounting member 4 for engagement in a recess 5 in a support block 6, and there secured by a screw 7 the head of which engages in a hole 8 in the block. On the block are mounted two parallel plates 9 each having an identical cam slot 10, emerging at the top edges of the plates, and where slotted locating members 11 are located.

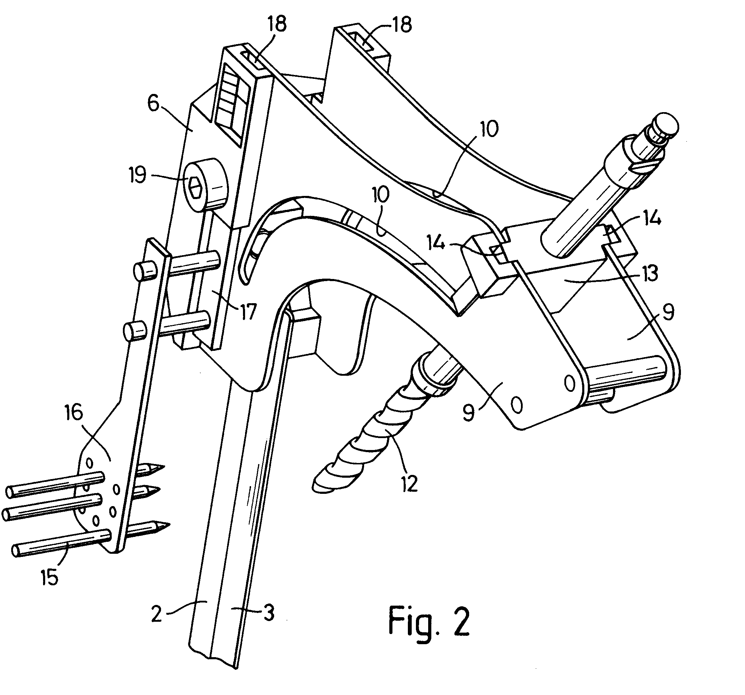

As is shown particularly by Figure 2, a fixing drill 12 is held in a required angular disposition by being slidably and rotatably mounted in a mounting member 13 having side ribs 14 that engage in the slots of the locating members. As is further shown by Figure 2, fixing pins 15 are provided on a plate 16, the plate 16 being secured by posts 17 engaging in slots 18 in the support block 6, and there secured by screws 19.

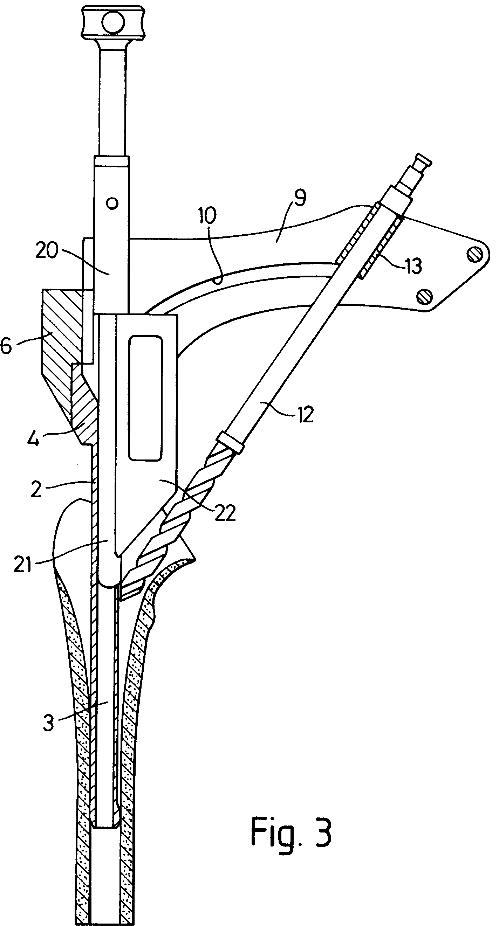

As is shown by Figure 3, an osteotome 20 is provided, having a rod 21 of a diameter to be a sliding fit in the tube 2, and having two blades 22 mounted on the rod, and having angled cutting surfaces at their lower ends.

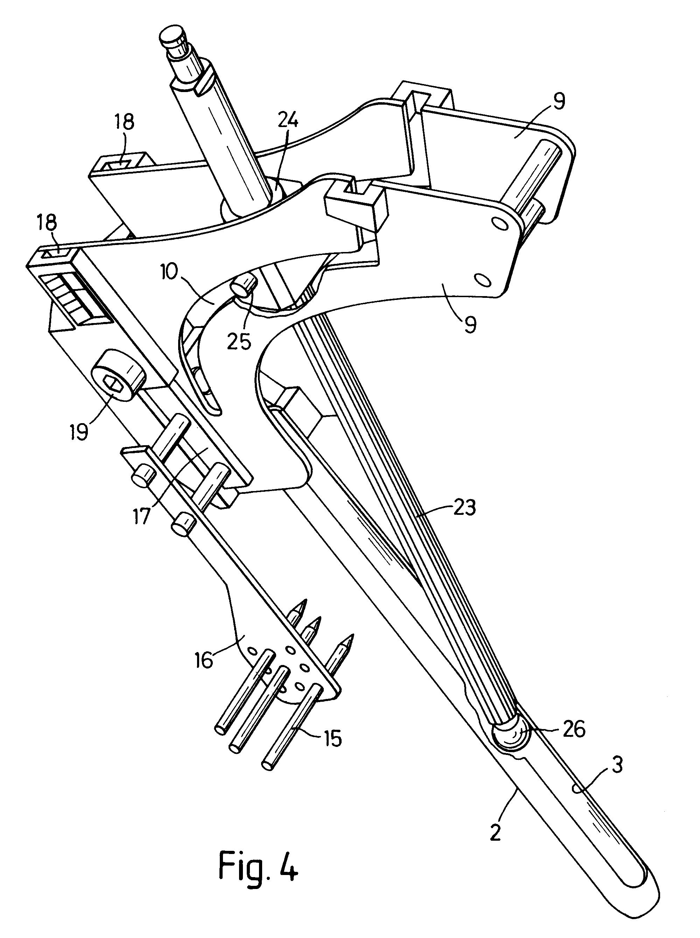

As shown by Figure 4, a reaming tool 23 is provided rotatively mounted in a bearing bush 24 having pins 25 to engage in the cam slots 10, and having a ball 26 of a diameter to be a sliding fit in the tube 2, but of a diameter greater than the slot 3, to be retained therein.

Thus, at the onset of an operation to replace a hip joint, the end of the femur is exposed in conventional manner, the femoral head disconnected from the hip socket, and the femoral head severed at the neck and removed. Following this, a hole is drilled down the femur centrally thereof and the tube 2 with its support block 6 located in the drilled hole. The fixing drill 12 is located on the plates 9 and drilled into the femoral neck, to secure the plates 9 in fixed spaced relationship to the femur. Once the drill is positioned within the neck of the femur, there is the substantial guarantee that the plates 9 and more particularly the cam slots 10 are so positioned that the subsequently formed slot (as is further described below) has an axis at a required angle to ensure that a subsequently positioned femoral component has the centre of its femoral head correctly disposed in its position for preservation of the biomechanics of the hip joint.

Following this, the fixing pins 15 are driven into the femur, to further secure the tube and support block in place and disposed as required.

With the drill in place, the osteotome rod is introduced into the tube 2, and the angled cutting surfaces of its blades 22 driven into the end of the femur to chop out a section of hard bone and form a start slot extending from the drilled hole. Following removal of the osteotome the drill is removed and, the reaming tool has its ball introduced down the tube 2, and the pins 25 of its bearing bush 24 located in the cam slots 10 of the plates 9, the ball 26 being urged down the tube to locate the end of the reaming tool in the start slot in the end of the femur. Rotation of the reamer and simultaneous progression of the bearing bush along the cam slots 10 results in the production of a parallel sided slot extending from the drilled hole, with a resultant medial surface at a precise and predetermined radius of curvature determined by cam slots 10, to correspond directly to radius of curvature of the medial flared surface of the femoral component of a replacement joint, to be received by the femur.

Once the slot has been cut, the pins 15 are removed from the femur, and the tube 2 with its support block and plates removed, and the femoral component introduced down the drilled hole in the femur, in the certain knowledge that the flared medial surface of the femoral component will fit exactly against the curved medial surface of the slot cut by the means according to the invention.

In hip prosthesis, two types of femoral component can be employed, one being a force fit in the hole and slot and the other being held in place by an appropriate cement. With a force fit component, the hole and slot formed as described above requires no further attention. However, with a cemented component, it is advisable to remove cancellous bone to the sides of the slot, to provide a slot that has its sides diverging upwardly from the bottom of the slot, in which the femoral component can sit with clearance and hence provide space for the cement.

## Claims

1. A bone removal means comprises a support means and a slotting means slidably mounted on said support means, characterised in that said slotting means (23) further engages a cam surface (10) located on said support means (2, 6, 9) and whereby simultaneous sliding movement of the slotting means (23) on said support means (2) and along said cam surface (10) combine to allow the cutting of a slot in the bone with a curved medial surface at a required predetermined radius of curvature.

**2.** A bone removal means as in Claim 1, characterised in that the slotting means is a rotatable reamer.

**3.** A bone removal means as in Claim 1 or Claim 2, characterised in that the support means comprises a tube (2) with a slot (3) to one side located on a support block (6) from which extend two plate members (9) each having a cam slot (10).

**4.** A bone removal means as in any of Claims 1 to 3, wherein location means (11) are provided on the support means (9), for the location of a fixing drill (12) at a required angular disposition, and whereby with the fixing drill driven into the femur neck, the cam surface (10) is set in a required disposition.

**6.** A bone removal means as in any of Claims 1 to 5, characterised by the provision of fixing pins (15) attached to the suppoort means (6), and whereby the support means (2, 6, 9) and particularly the cam surface (10) is secured in fixed and required position on the femur.

**7.** A bone removal means as in any of Claims 1 to 6, characterised in that an osteotome (20) is provided, to generate a start slot in the exposed end of the femur, for subsequent engagement and enlargement by said slotting tool (23).

Fig. 1

Fig. 2

Fig. 3

Fig. 4

European Patent
Office

**EUROPEAN SEARCH REPORT**

Application Number

EP    92 30 0628

| DOCUMENTS CONSIDERED TO BE RELEVANT |||||
|---|---|---|---|---|
| Category | Citation of document with indication, where appropriate, of relevant passages | | Relevant to claim | CLASSIFICATION OF THE APPLICATION (Int. Cl.5) |
| Y | EP-A-0 263 292 (SULZER) * column 2, line 2 - line 48; figure 1 * --- | | 1-6 | A61F2/46 |
| Y | DE-A-3 522 971 (EISENBACH) * column 3, line 45 - column 4, line 24; figure 1 * --- | | 1-6 | |
| A | CH-A-560 042 (ROSENTHAL STEMAG) * column 2, line 37 - line 59; figures 5-7 * --- | | 4 | |
| A | EP-A-0 337 901 (BROC) * column 3, line 17 - line 21; figure 1 * --- | | 5 | |
| A | WO-A-8 702 571 (ROGER) * page 6, line 29 - page 7, line 9; figure 7 * ----- | | 1 | |
| | | | | **TECHNICAL FIELDS SEARCHED (Int. Cl.5)** |
| | | | | A61F A61B B23Q |
| The present search report has been drawn up for all claims ||||
| Place of search | Date of completion of the search | | Examiner ||
| THE HAGUE | 23 APRIL 1992 | | MOERS R. ||

EPO FORM 1503 03.82 (P0401)